# FASCICULE DE BREVET EUROPEEN

(11) **EP 3 727 217 B1**
(45) Date de publication et mention de la délivrance du brevet: **17.09.2025**
(21) Numéro de dépôt: 18833472.6
(22) Date de dépôt: 12.12.2018
(51) Int. Cl.: A61F 5/01

(54) **ORTHESE DE REPOS D'UNE ARTICULATION**
ORTHESE ZUR IMMOBILISIERUNG EINES GELENKS
ORTHOSIS FOR IMMOBILIZING A JOINT

(30) Priorité: 21.12.2017 FR 1762847
(43) Date de publication de la demande: 28.10.2020
(73) Titulaire: Millet Innovation, 26270 Loriol sur Drome (FR)
(72) Inventeur: MILLET, Damien, 26000 Valence (FR); LAURENT, Hugo, 26000 Valence (FR); GRANGE, Odile, 26400 Allex (FR); TREPIER-LE BELLER, Maria Luisa, 26800 Portes-les-Valence (FR); MARTIN, Océane, 26270 Loriol sur Drome (FR); FONTAINE, Thierry, 26740 Marsanne (FR)
(74) Mandataire: Marchand, André
(86) Numéro de dépôt international: PCT/FR2018/053227
(87) Numéro de publication internationale: WO 2019/122610

(56) Documents cités:
- EP-A2- 2 090 273
- US-A- 5 397 296
- US-A- 5 807 293
- US-A1- 2005 165 338
- US-A1- 2010 081 981
- US-A1- 2017 354 528
- US-B1- 6 716 185

## Description

La présente invention est définie dans la revendication 1 et concerne une orthèse de repos d'une articulation et plus particulièrement d'une articulation du pouce ou du poignet.

Depuis de nombreuses années, on a développé des orthèses visant des pathologies accidentelles, puis, plus récemment des pathologies liées au vieillissement. Dans le cas des pathologies accidentelles, on cherche en général à immobiliser un membre ou une articulation. Dans le cas de pathologies liées au vieillissement ou de pathologies accidentelles bénignes, on cherche plutôt à assurer un simple maintien du membre ou de l'articulation, en l'absence de sollicitation musculaire, sans empêcher les mouvements volontaires.

La plupart des orthèses adaptées à un membre ou une articulation sont réalisées par l'association de tissus tricotés et d'éléments rigides par exemple réalisés en acier ou en matière plastique. Ces orthèses présentent les inconvénients d'être relativement inconfortables notamment en raison de leur poids et leur encombrement. Ces orthèses apparaissent donc inadaptées au simple maintien d'un membre ou d'une articulation pendant de longues périodes.

Dans le cas de personnes atteintes de rhizarthrose ou d'une tendinite de Quervain, il est généralement prescrit de mettre au repos l'articulation trapézo-métacarpienne du pouce, afin de prévenir la détérioration des symptômes à long terme et de soulager la douleur. Or, même si le pouce n'est pas sciemment utilisé, son poids provoque de lui-même une activité sur cette articulation.

Le Demandeur a développé des orthèses plus confortables et légères, décrites dans la demande de brevet WO 2015/28734 (US 2016/206464). Ces orthèses comprennent une pièce en tissu entourant l'articulation du pouce, dont la rigidité est accrue par un effet de poutre, pour prévenir les mouvements involontaires, sans imposer des efforts supplémentaires pour effectuer des mouvements volontaires.

Cependant, d'autres pathologies peuvent motiver une limitation des mouvements de l'une ou l'autre des articulations de main et du poignet. Ainsi, dans le cas d'un syndrome de canal carpien, un mouvement de flexion répété du poignet peut entrainer des douleurs. Or à l'état de repos, le seul poids de la main provoque souvent une flexion involontaire du poignet. Le maintien du poignet en position de repos peut donc être préconisé notamment pendant le sommeil. Egalement, dans le cas de troubles musculo-squelettiques impactant le pouce et le poignet, il peut être préconisé de maintenir toutes les articulations concernées au repos. Il s'avère que la pièce de tissu utilisée pour maintenir le pouce présente une rigidité insuffisante, même en faisant appel à un effet de poutre, pour supporter le poids de la main et ainsi maintenir le poignet.

Le Demandeur a également proposé des orthèses intégrant des plaquettes rigides thermoformables, décrites dans la demande de brevet WO 2016/198778. Ces orthèses permettent d'assurer un maintien très efficace, adapté à la morphologie de l'usager. Cependant, ce maintien peut apparaître excessif pour certaines pathologies, notamment la nuit, et ainsi être source d'inconfort. Ces orthèses présentent également l'inconvénient de nécessiter une opération de thermoformage. Par ailleurs, il peut être nécessaire d'éviter toute pression dans une zone déterminée. C'est le cas notamment des personnes souffrant de pathologies du canal carpien. L'opération de thermoformage nécessite alors l'emploi d'une pièce à disposer sur la zone à protéger de toute pression pendant l'opération de thermoformage, de manière à ménager un espace entre cette zone et la plaquette thermoformable, l'orthèse étant ensuite utilisée sans cette pièce.

La demande de brevet EP 2 090 273 décrit des orthèses de poignet comportant un manchon recouvrant le poignet, la main et une partie du pouce, et une plaquette maintenue sur le manchon dans une poche ou par une bande centrée sur la plaquette et serrée autour du poignet.

Il est donc souhaitable de réaliser une orthèse de repos d'une articulation, telle que celles de la main et/ou du poignet, capable de supporter le poids de la partie distale du membre à partir de l'articulation, sans être source de gêne, même si l'orthèse est portée pendant de longues périodes. Il peut être également souhaitable de réaliser une orthèse qui ne nécessite pas d'être réalisée sur mesure, mais seulement en quelques tailles standard, et sans nécessiter d'opération d'ajustement final. Il peut être également souhaitable de réaliser une orthèse qui n'entrave pas les mouvements de l'articulation.

Des modes de réalisation concernent une orthèse pour le maintien d'une articulation, adaptée au pouce et/ou au poignet, et comprenant : une pièce principale en forme de manchon en un matériau élastique, et ajustée pour envelopper l'articulation et des parties de membre distale et proximale de part et d'autre de l'articulation, la pièce principale comportant une ouverture pour le passage d'une partie proximale de membre et une ouverture pour le passage d'une partie distale de membre, une plaquette déformable élastiquement logée dans une poche formée dans la pièce principale et ajustée aux dimensions de la plaquette, une partie distale de la plaquette étant maintenue autour du membre seulement par la pièce principale, la plaquette étant configurée pour que, sur au moins 50% de la longueur de la pièce principale le long des parties de membre, la plaquette présente une section occupant plus de 30% du périmètre du membre sous-jacent. Selon l'invention, l'orthèse comprend un dispositif de serrage non élastique de la pièce principale dans la région d'au moins 50% de la longueur de la pièce principale, et d'une partie proximale de la plaquette autour de la partie de membre proximale, la plaquette présentant une rigidité correspondant à un module de Young compris entre 5 à 10 MPa, tandis que le matériau formant la partie principale présente une rigidité d'un ordre de grandeur inférieur.

Selon un mode de réalisation, l'étendue et l'épaisseur de la plaquette, et l'élasticité de la plaquette et de la partie principale sont choisies de manière à empêcher des mouvements involontaires de l'articulation à partir d'une position de repos neutre, provoqués par le poids de la partie distale de membre.

Selon un mode de réalisation, la plaquette s'étend sur au moins 80% de la longueur de l'orthèse le long du membre, et la section de la plaquette occupe plus de 40% du périmètre du membre sous-jacent sur au moins 80% de la longueur de la plaquette le long du membre.

Selon un mode de réalisation, la plaquette comprend deux parties de plaquette liées entre elles par une charnière s'étendant le long d'une partie de bords en regard des deux parties de plaquette.

Selon un mode de réalisation, la charnière est réalisée lors de la fabrication de la plaquette dans le même matériau que la plaquette, ou bien est formée par des points de couture.

Selon un mode de réalisation, la charnière s'étend sur moins la moitié de la longueur des bords en regard des deux parties de plaquette.

Selon un mode de réalisation, le dispositif de serrage couvre en partie la charnière.

Selon un mode de réalisation, l'orthèse comprend une autre plaquette, les deux plaquettes étant logées dans des poches distinctes et contigües.

Selon un mode de réalisation, la pièce principale est réalisée dans une pièce de tissu formée de deux couches de tissu élastique assemblées par une couche de colle, chaque poche étant formée à l'aide d'une couche de tissu élastique cousue sur une face intérieure de la pièce principale.

Selon un mode de réalisation, la plaquette est réalisée dans un matériau présentant un module de Young compris entre 5,4 et 9 MPa, et présente une épaisseur comprise entre 0,8 et 1,9 mm.

Selon un mode de réalisation, l'orthèse est adaptée au maintien du pouce, la pièce principale comportant une partie distale en forme de manchon conformé pour couvrir la base du pouce, une partie proximale en forme de manchon conformé pour couvrir le poignet, et une partie intermédiaire reliant les parties distale et proximales, la plaquette comprenant deux parties de plaquettes conformées pour épouser la forme du pouce, de la première phalange et du premier métacarpien du pouce, jusqu'au poignet, sans recouvrir la zone palmaire du poignet, le dispositif de serrage comprenant une sangle fixée à la pièce principale et agencée pour être serrée autour du poignet.

Selon un mode de réalisation, l'orthèse est adaptée au maintien du poignet, la pièce principale en forme de manchon, est conformée pour couvrir une partie de l'avant-bras, le poignet et les articulations carpo-métacarpiennes du pouce, et une partie proximale des métacarpes des doigts, la pièce principale présentant une ouverture proximale pour le passage de l'avant-bras, et deux ouvertures distales, l'une pour le passage du pouce, et l'autre pour le passage de la paume de la main, la plaquette comprenant deux parties de plaquettes conformées pour épouser la base de la paume de la main, la région du métacarpien du pouce, et la face palmaire du poignet, et d'une partie de l'avant-bras, en remontant sur les faces latérales de la main, du poignet et de l'avant-bras, le dispositif de serrage étant agencé pour serrer la pièce principale autour du poignet et de l'avant-bras.

Selon un mode de réalisation, l'orthèse est adaptée au maintien du poignet et du pouce, la pièce principale comprend une partie distale en forme de manchon conformé pour couvrir la base du pouce, une partie proximale en forme de manchon conformé pour couvrir le poignet et une partie de l'avant-bras et les articulations carpo-métacarpiennes du pouce, et une partie intermédiaire reliant les parties distale et proximale, la plaquette comprenant deux parties de plaquettes conformées pour épouser la forme du pouce, de la première phalange et de la région du premier métacarpien du pouce, la base de la paume de la main, et la face palmaire du poignet, et d'une partie de l'avant-bras, en remontant sur les faces latérales de la main, du poignet et de l'avant-bras, le dispositif de serrage étant agencé pour serrer la pièce principale autour du poignet et de l'avant-bras.

Selon un mode de réalisation, l'orthèse est l'une des deux parties de plaquettes est conformée pour ne pas serrer la région du canal carpien sur la face palmaire du poignet.

Des exemples de réalisation de l'invention seront décrits dans ce qui suit, à titre non limitatif en relation avec les figures jointes parmi lesquelles :
la figure 1 représente schématiquement en coupe une partie d'orthèse selon un mode de réalisation,
les figures 2 à 4 sont des vues schématiques en coupe de plaquettes moulées, souples, insérées dans une poche formée dans l'orthèse, selon divers modes de réalisation,
les figures 5 et 6 sont des vues schématiques en perspective de paires de plaquettes liées entre elles par une articulation, selon différents modes de réalisation,
les figures 7 et 8 sont des vues schématiques opposées d'une main équipée d'une orthèse de pouce selon un mode de réalisation,
les figures 9 et 9A représentent deux faces opposées d'une paire de plaquettes équipant l'orthèse de la figure 7, respectivement en vue de face et en perspective, selon un mode de réalisation,
la figure 10 est une vue schématique de l'orthèse de la figure 8, illustrant un effet obtenu par la paire de plaquettes de la figure 9, selon un mode de réalisation,
les figures 11 et 12 sont des vues schématiques opposées d'une main équipée d'une orthèse de poignet selon un mode de réalisation,
La figure 13 représente schématiquement un dispositif de serrage pour l'orthèse de la figure 11, selon un mode de réalisation,
La figure 14 représente schématiquement l'orthèse de la figure 11 équipée d'une sangle de serrage selon un autre mode de réalisation,
la figure 15 représente schématiquement une paire de plaquettes équipant l'orthèse de la figure 11, selon un mode de réalisation,
la figure 16 est une vue latérale schématique de l'orthèse de la figure 11, illustrant un effet obtenu par la paire de plaquettes de la figure 15, selon un mode de réalisation,
la figure 17 représente schématiquement une paire de plaquettes équipant une orthèse de poignet et de pouce, selon un mode de réalisation,
la figure 18 est une vue latérale schématique d'une orthèse de poignet et de pouce, selon un mode de réalisation.

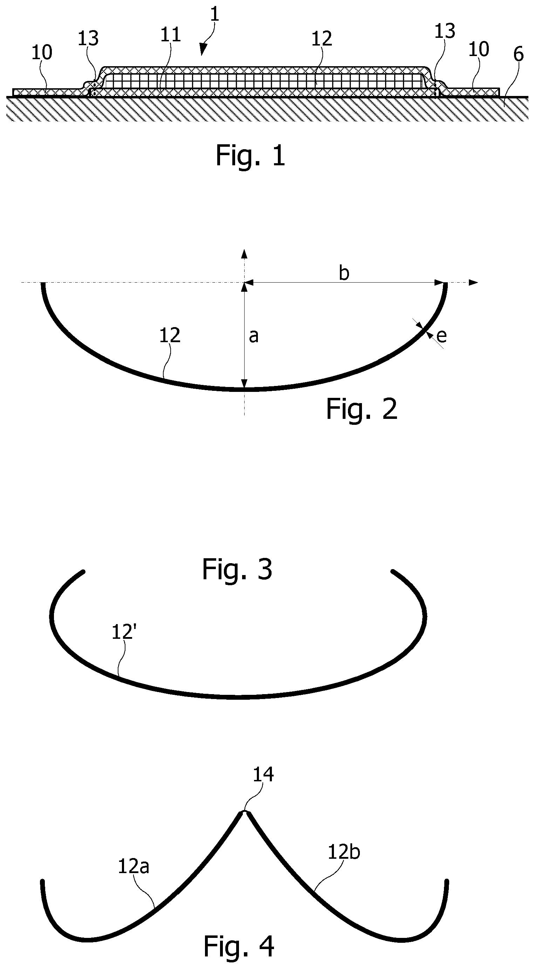

La figure 1 représente une partie d'orthèse 1 selon un mode de réalisation. L'orthèse 1 comprend deux pièces 10, 11. La pièce 10 est au moins en partie élastique, et conformée de manière à former un manchon ajusté autour d'une articulation à maintenir. Ainsi, le manchon peut exercer sur l'articulation et/ou le membre sous-jacent une tension comparable ou inférieure à celle exercée par les dispositifs de contention les plus fréquemment utilisés. La pièce 11 est réalisée dans un matériau élastique et fixée sur la pièce 10 de manière à former une poche dans laquelle est insérée une plaquette 12, en un matériau déformable élastiquement. La forme de la poche est ajustée à celle de la plaquette 12. Ainsi le taux de remplissage de la poche par la plaquette peut être compris entre 80 et 95%. La forme de la plaquette 12 peut être elle-même définie en fonction d'une zone du membre ou de l'articulation où le maintien est à assurer. La pièce 10 peut être mise en contact direct avec la peau 6 du membre. La pièce 11 peut être fixée sur la pièce 10 par une ligne de soudure ou de couture 13, sur la face de la pièce 10 en contact avec la peau 6. La pièce 11 est donc également en contact avec la peau 6 du membre.

Selon un mode de réalisation, la plaquette 12 est conformée de manière à envelopper et épouser, au moins partiellement la forme de la partie de l'articulation sur laquelle elle est destinée à être appliquée. Ainsi, la plaquette 12 peut être conformée de manière à envelopper partiellement l'articulation en s'appliquant contre des zones à maintenir de l'articulation, et en ménageant un espace entre l'orthèse et la peau du membre dans des zones sensibles où il est préférable de ne pas exercer de pression.

L'orthèse comprend également un dispositif de serrage non élastique, de la pièce principale et d'une partie proximale de la plaquette autour de la partie de membre proximale. Ainsi, la plaquette combinée au dispositif de serrage permet de former une poutre encastrée, lorsque le dispositif de serrage est ajusté autour du membre, y compris à une tension nulle ou faible, qui permet simplement d'ajuster la longueur du dispositif de serrage au périmètre du membre sous-jacent. La plaquette est également configurée pour que, sur au moins 50% de la longueur de l'orthèse le long des parties de membres couvertes par l'orthèse, incluant la partie de membre couverte par le dispositif de serrage, la plaquette présente une section occupant plus de 30% du périmètre du membre sous-jacent. Par ailleurs, l'étendue et l'épaisseur de la plaquette, et l'élasticité de la plaquette et de la partie principale sont choisies de manière à empêcher des mouvements involontaires de l'articulation à partir d'une position de repos neutre, liés au poids de la partie distale du membre. Il en résulte que quelle que soit l'orientation de la partie distale du membre, l'orthèse peut assurer seule le maintien de cette partie dans sa position neutre,

De préférence, la plaquette s'étend sur au moins 80% de la longueur de l'orthèse le long du membre, et la section de la plaquette occupe plus de 40% du périmètre du membre sous-jacent sur au moins 80% de la longueur de la plaquette le long du membre.

Selon un mode de réalisation, l'étendue et l'épaisseur de la plaquette, ainsi que l'élasticité de la plaquette et de la partie principale sont choisies de manière à autoriser des mouvements volontaires de l'articulation.

La plaquette 12 peut être réalisée par moulage en une matière plastique injectable, ou par thermoformage d'une plaque plane à l'aide d'une presse à chaud, ou encore par impression 3D. La plaquette 12 peut présenter une épaisseur variable de manière à être plus épaisse dans des zones où la plaquette 12 doit être plus rigide pour assurer un certain maintien, et moins épaisse dans des zones où la plaquette doit être plus souple pour assurer un maintien moins ferme ou pour former une charnière entre deux parties de la plaquette. L'élasticité du matériau (module d'Young) formant la plaquette 12 est choisie en fonction de la rigidité recherchée et de la forme, et en particulier de la courbure, de la plaquette.

La figure 2 représente un exemple de section de plaquette 12 susceptible d'équiper l'orthèse selon l'invention. La section de plaquette représentée sur la figure 2 présente une forme semi-elliptique, de profondeur a, de demi-largeur b et d'épaisseur e. La flexion d'une poutre ayant cette section est inversement proportionnelle au module d'Young, au cube de la profondeur a et à la demi-largeur b. Bien entendu, la forme de section de plaquette représentée sur la figure 2 est une forme idéale qui ne correspond pas à la section d'une articulation ou d'un membre.

Dans certains cas, il peut être souhaitable que la plaquette entoure localement davantage l'articulation. Ainsi, la figure 3 représente une section de plaquette 12' s'étendant sur un secteur angulaire supérieur à 180°. Il apparaît que la plaquette 12' ne peut pas être réalisée à l'aide d'un moule simple sans contre dépouille. Selon un mode de réalisation, une telle plaquette est réalisée en deux parties comme illustré sur la figure 4. La figure 4 représente les sections d'une paire de plaquettes 12a, 12b qui peuvent être assemblées par une charnière 14 pour former une plaquette ayant la section de la plaquette 12'. La paire de plaquettes 12a, 12b peut être réalisée par une seule opération de moulage à l'aide d'un moule simple (sans contre dépouille).

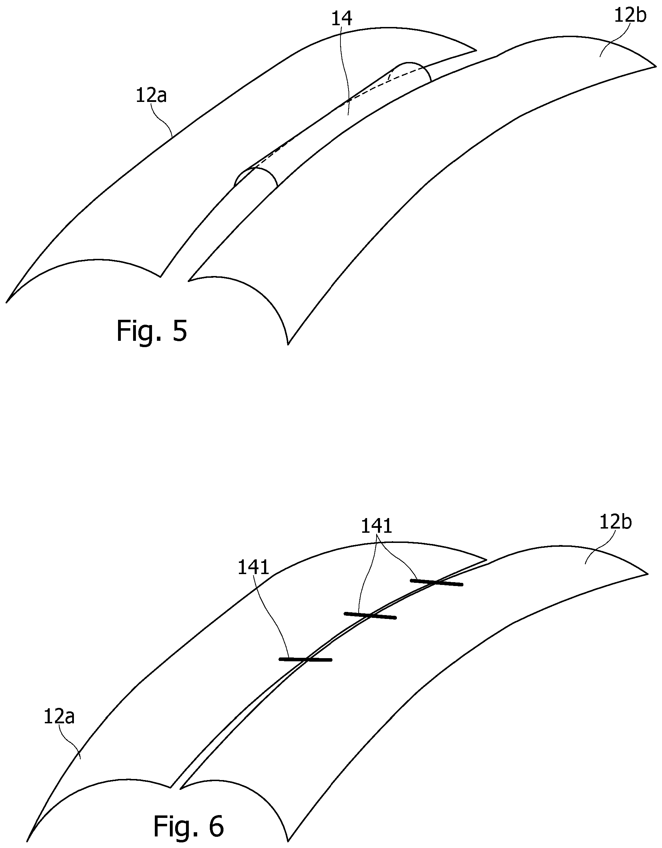

Les figures 5 et 6 représentent des exemples de paires de plaquettes 12a, 12b assemblées par une charnière. Dans l'exemple de la figure 5, la charnière 14 est formée en même temps que les plaquettes 12a, 12b par l'opération de moulage, et donc dans le même matériau que les plaquettes. La charnière 14 présente une forme sensiblement cylindrique (définie par une génératrice rectiligne) et peut présenter une épaisseur moins élevée que celle des plaquettes 12a, 12b, pour permettre son pliage par déformation élastique. En effet, pour assurer sa fonction, la charnière 14 ne peut pas être courbe, même si les plaquettes 12a, 12b sont courbes pour épouser une partie de membre.

Dans l'exemple de la figure 6, la charnière est réalisée par des points de couture 141, par exemple 3 points de couture.

Selon un mode de réalisation, la charnière 14, 141 s'étend sur une partie seulement de bords en regard des deux plaquettes 12a, 12b. En effet, on observe peu de variations de dimension des articulations entre les individus adultes. En revanche, d'importantes variations morphologiques de dimension peuvent être observées dans les régions de part et d'autre des articulations. Il est donc avantageux de ne pas lier les plaquettes 12a, 12b par une charnière sur une partie des bords en regard des deux plaquettes 12a, 12b. Grâce à la souplesse des plaquettes 12a, 12b, celles-ci peuvent se déformer et se conformer à la morphologie de l'utilisateur, dans les zones non liées par la charnière. En revanche, dans les zones liées par la charnière, l'ensemble des deux plaquettes 12a, 12b présente une rigidité proche de celle d'une plaquette unique ayant la forme des deux plaquettes 12a, 12b liées l'une à l'autre.

Selon un autre mode de réalisation, les deux plaquettes 12a, 12b sont simplement placées dans des poches respectives de l'orthèse sans être liées par une charnière, notamment dans le cas où une rigidité moindre est requise, et/ou lorsque le matériau choisi pour les plaquettes présente une rigidité suffisante. Les deux plaquettes 12a, 12b peuvent également être placées dans une poche unique séparée en deux par exemple par une couture réalisée dans un intervalle séparant des bords en regard des deux plaquettes.

Selon un mode de réalisation, le matériau dans lequel la pièce 10 est formée, est réalisé à l'aide de deux couches d'un tissu élastique, assemblées l'une sur l'autre par une couche de colle. La couche de colle peut être uniformément répartie entre les deux couches de tissu, ou bien disposée par points uniformément répartis. Les deux couches de tissu peuvent être collées l'une contre l'autre en enduisant de colle l'une des deux couches, et en pressant les deux couches l'une contre l'autre, au moyen d'un ou deux rouleaux. La couche de colle peut présenter une épaisseur d'environ 0,05 mm.

Le tissu formant les deux couches de la pièce 10 peut être un tissu à base de polyamide (environ 80% en poids) et d'élasthanne (environ 20% en poids), ayant un grammage de 155 g/m2. La colle utilisée peut être à base de polyuréthane. L'ensemble du matériau formé par les deux épaisseurs de tissu et de la couche de colle peut présenter un grammage de 355 g/m2. Le tissu formant les deux couches peut présenter une épaisseur comprise entre 0,5 et 0,7 mm. Il en résulte que le matériau dans lequel la pièce 10 est formée peut présenter une épaisseur comprise entre 1 et 1,4 mm. Le tissu formant chacune des deux couches collées peut présenter une élasticité comprise entre 85% à 115% dans le sens de la chaine et de 65% à 95% dans le sens de la trame. La pièce 11 assemblée sur la couche 10 pour former la poche peut être réalisée par une seule couche de ce même tissu.

Pour limiter les risques de sudation, les pièces 10 et 11 peuvent également présenter des micro-perforations.

Selon un exemple de réalisation, la ou les plaquettes peuvent être réalisées en un élastomère thermoplastique, et peuvent présenter un module de Young (en l'absence d'étirement) compris entre 5 à 10 MPa pour une épaisseur comprise entre 0,7 et 2 mm. De préférence, le module de Young de la ou les plaquettes est compris entre 5,4 et 9 MPa pour une épaisseur comprise entre 0,8 et 1,9 mm.

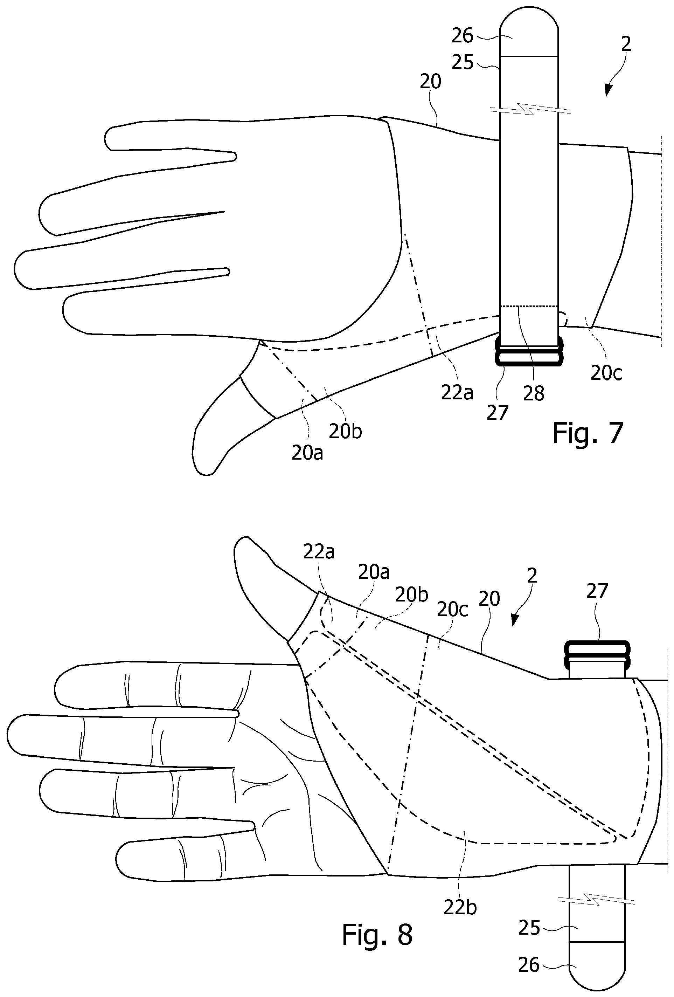

Les figures 7 et 8 représentent une orthèse de pouce 2 selon un mode de réalisation, l'orthèse étant placée sur une main. Les figures 7 et 8 représentent respectivement la face dorsale et la face palmaire de la main. L'orthèse 2 comprend une pièce principale 20 comportant une partie distale 20a, une partie proximale 20c, et une partie intermédiaire 20b reliant les parties 20a et 20c, délimitées sur les figures 7 et 8 par des lignes en trait mixte à points et tirets. La partie 20a, en forme de manchon, est conformée pour couvrir et maintenir la base du pouce, de la zone de pli entre le pouce et la paume de la main, jusqu'au milieu de la première phalange du pouce. La partie 20c, également en forme de manchon, est conformée pour couvrir le poignet, les articulations carpo-métacarpiennes du pouce, et une partie proximale des métacarpes des doigts. La partie intermédiaire 20c présente une ouverture s'étendant entre les parties 20a et 20c, pour le passage de la paume de la main.

La pièce principale 20 est réalisée dans un matériau élastique et ajustée à la forme de la main et du pouce à maintenir, sans y exercer de force de contention trop intense, susceptible de provoquer des douleurs, en particulier dans la zone sensible des articulations carpo-métacarpiennes. La pièce principale 20 peut ainsi être réalisée dans le même matériau que la pièce 10 (Figure 1). L'orthèse comprend également une sangle de serrage 25 non élastique, disposée de manière à serrer la pièce principale 20 autour du poignet. La sangle 25 peut être fixée à la pièce principale 20, par exemple par une couture 28. Le serrage de la sangle peut être ajusté au moyen d'une bande à boucles recouvrant une face de la sangle et une plaquette à crochets 26 fixée à l'extrémité libre de la sangle. Dans un exemple de réalisation une extrémité de la sangle est équipée d'une boucle de serrage 27 dans laquelle l'autre extrémité de la sangle est engagée après avoir été enroulée dans un sens autour du poignet. La sangle est ensuite serrée autour du poignet dans l'autre sens, la partie à crochets 26 à l'autre extrémité de la sangle étant appliquée sur la bande à boucles sur la sangle. L'orthèse peut s'enlever en direction de l'extrémité des doigts et du pouce, sans effort important, notamment en raison de l'absence de zones d'étranglement.

Selon un mode de réalisation, la pièce principale 20 comprend une poche dans laquelle sont insérées des plaquettes 22a, 22b liées entre elles par une ou plusieurs charnières. Les plaquettes 22a, 22b sont moulées de manière à épouser la forme du pouce, de la première phalange et du premier métacarpien, jusqu'au poignet. Ainsi, une partie proximale des plaquettes 22a, 22b se trouve dans la zone serrée par la sangle 25.

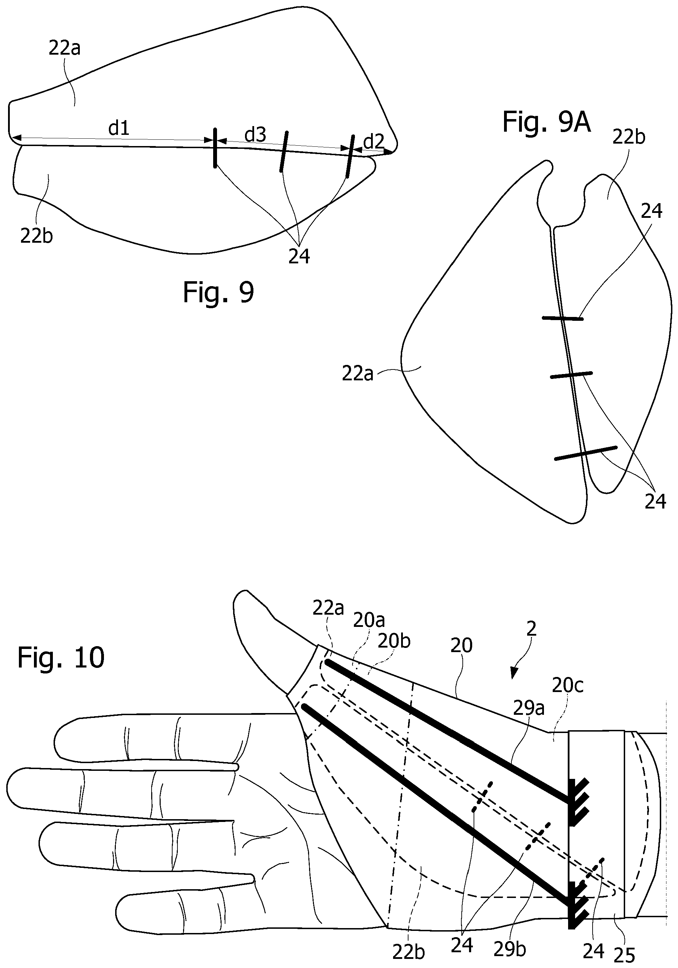

Les figures 9 et 9A représentent les plaquettes 22a, 22b liées entre elles par trois coutures 24. Les plaquettes 22a, 22b présentent ensemble une forme enveloppante, en entourant plus de 80% de la circonférence de la première phalange. Cette forme enveloppante présente une profondeur (cote "a" sur la figure 2) qui contribue à augmenter le moment quadratique des plaquettes, et donc, par effet de poutre à augmenter la rigidité des plaquettes pour supporter le poids du pouce. Comme illustré sur la figure 10, une partie des plaquettes se trouve sous la sangle 25. Les plaquettes 22a, 22b se comportent ainsi comme des poutres encastrées 29a, 29b, lorsque la sangle 25 est serrée autour du poignet, le serrage par la sangle 25 d'une partie des plaquettes 22a, 22b produisant un effet d'encastrement. Cet effet d'encastrement est augmenté par le fait que la sangle n'est pas élastique. Ainsi, la forme enveloppante des plaquettes 22a, 22b, associée à la rigidité du matériau choisi pour former les plaquettes, permet d'assurer un maintien de l'articulation métacarpo-phalangienne du pouce en position de repos. La rigidité du matériau formant les plaquettes est choisie de manière à empêcher des mouvements involontaires de l'articulation à partir de la position de repos neutre du pouce (en l'absence de force externe et de contraction musculaire), liés au poids du pouce, quelle que soit l'orientation de la main dans l'espace, sans toutefois empêcher les mouvements volontaires du pouce.

Il peut être observé sur la figure 10 qu'une partie de la charnière 24 se trouve couverte par la sangle de serrage 25, ce qui contribue à la rigidité de l'orthèse. Il peut également être observé que la charnière 24 ne s'étend que sur une partie des bords en regard des deux plaquettes 22a, 22b. Une partie distale d1 (figure 9) sans charnière permet à l'orthèse de s'adapter aux différentes morphologies de la région de l'articulation métacarpo-phalangienne du pouce. En effet, l'absence de charnière dans cette zone permet aux plaquettes de s'écarter ou se rapprocher l'une de l'autre, ainsi que de se déformer de façon plus importante que si ces parties étaient liées. Cette disposition combinée à la souplesse des plaquettes 22a, 22b permet d'utiliser les mêmes plaquettes pour différentes tailles de l'orthèse, et ainsi d'utiliser un seul moule pour la fabrication d'orthèses de différentes tailles. La présence de la charnière 24 liant les deux plaquettes 22a, 22b dans la zone d3 permet de former un profil combiné plus profond (cote "a" sur la figure 2). Le moment quadratique obtenu par la combinaison des deux plaquettes est donc légèrement plus élevé dans cette zone.

Selon un mode de réalisation, la distance d1 est fixée à une valeur comprise entre 60 et 65 mm, la longueur d3 de la charnière est fixée à une valeur comprise entre 35 et 45 mm, et la distance d2 entre la charnière et le bord proximal de la plaquette 22a est fixée à une valeur comprise entre 10 et 15 mm.

Selon un autre mode de réalisation, les plaquettes 22a, 22b ne sont pas liées entre elles par une articulation, mais placées dans deux poches distinctes ou une seule poche divisée en deux, par exemple par une couture de séparation entre les deux plaquettes.

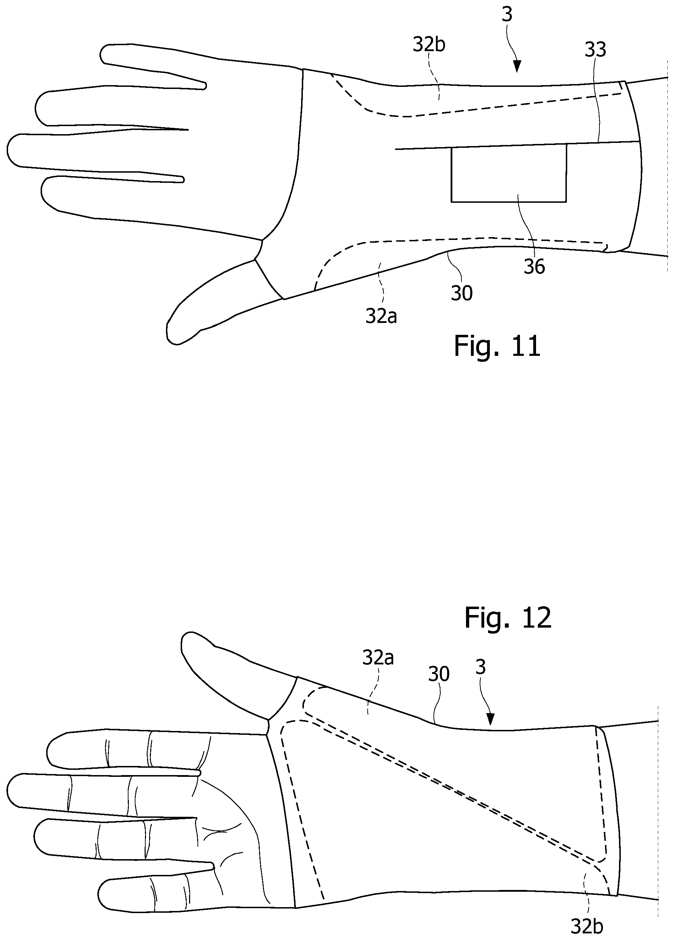

Les figures 11 et 12 représentent une orthèse de poignet 3, selon un mode de réalisation, l'orthèse étant placée sur une main. Les figures 11 et 12 représentent respectivement la face dorsale et la face palmaire de la main. L'orthèse 3 comprend une pièce principale 30 en forme de manchon, couvrant une partie de l'avant-bras, le poignet et les articulations carpo-métacarpiennes du pouce, et une partie proximale des métacarpes des doigts. La pièce principale 30 présente une ouverture proximale pour le passage de l'avant-bras, et deux ouvertures distales, l'une pour le passage du pouce, et l'autre pour le passage de la paume de la main. La pièce principale 30 comporte une fente 33 s'étendant de l'ouverture proximale jusqu'à une région centrale de la face dorsale de la main, pour permettre le passage de la main dans l'orthèse. La fente 33 peut être associée à un dispositif de fixation, par exemple un dispositif à boucles et à crochets 36, permettant également d'ajuster le positionnement de l'orthèse autour de l'avant-bras.

Selon un mode de réalisation, la pièce principale 30 comprend une poche dans laquelle sont insérées des plaquettes 32a, 32b liées entre elles par une ou plusieurs charnières. Les plaquettes 32a, 32b sont conformées de manière à épouser la base de la paume de la main, la région du métacarpien du pouce, la face palmaire du poignet, et une partie de l'avant-bras, en remontant sur les faces latérales de la main, du poignet et de l'avant-bras. Les plaquettes 32a, 32b sont également conformées de manière à ne pas venir en contact avec la région du canal carpien de la face palmaire du poignet, qui peut être sensible, notamment en cas de syndrome du canal carpien.

La pièce principale 30 est réalisée dans un matériau élastique et ajustée à la forme de la main et du pouce à maintenir, sans y exercer de force de contention trop intense, susceptible de provoquer des douleurs, en particulier dans la zone sensible des articulations carpo-métacarpiennes. La pièce principale 30 peut ainsi être réalisée dans le même matériau que la pièce 10 (Figure 1). L'orthèse comprend également un dispositif de serrage 35 non élastique, disposée de manière à serrer la pièce principale 30 autour du poignet et de l'avant-bras.

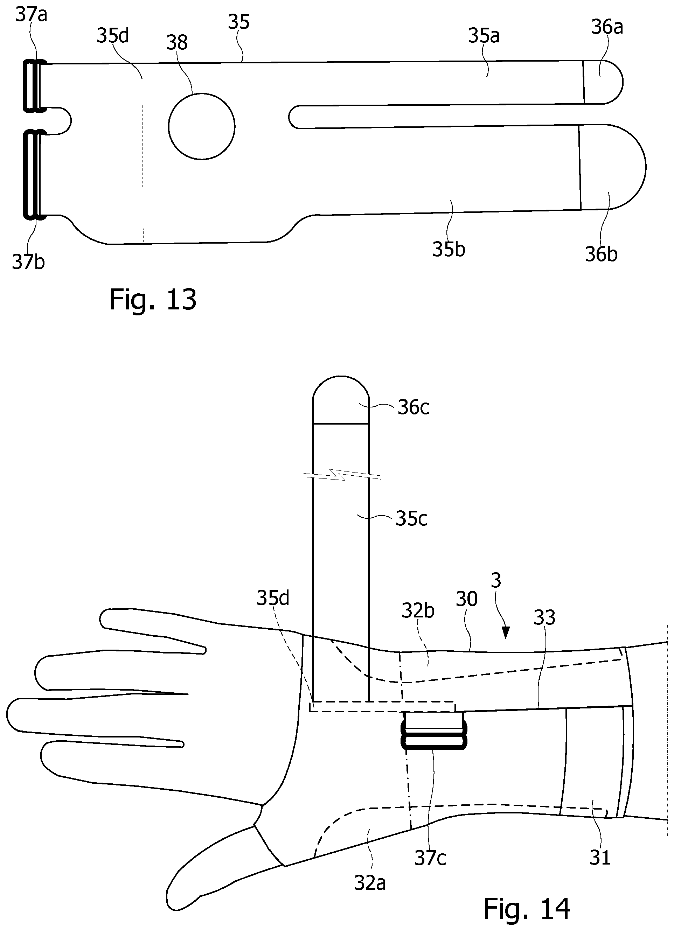

La figure 13 représente un exemple de réalisation d'un dispositif de serrage 35 de l'orthèse 3. Le dispositif de serrage 35 comprend une partie principale sensiblement rectangulaire. Cette partie principale peut être munie d'une ouverture 38 pour éviter de couvrir la région de la styloïde, plus sensible en raison de sa proéminence. L'ouverture 38 peut également constituer un repère pour faciliter le positionnement du dispositif de serrage autour du poignet. Les bords latéraux de cette partie principale sont prolongés d'un côté par des parties de sangle 35a, 35b, et de l'autre par des parties de sangle dont les extrémités sont équipées de boucles de serrage 37a, 37b prévues pour coopérer avec les parties de sangle 35a, 35b. Les parties de sangle 35a, 35b peuvent être de largeurs différentes. Une face du dispositif de serrage 35 peut être recouverte d'une bande à boucles destinée à coopérer avec des plaquettes à crochets 36a, 36b par exemple disposées aux extrémités libres des parties de sangles 35a, 35b, pour assurer le serrage du dispositif 35 autour de l'orthèse 3. Le dispositif de serrage 35 peut être fixé à la partie principale 30 par exemple le long de la fente 33, au moyen d'une couture 35d formée entre l'ouverture 38 et les boucles de serrage 37a, 37b.

Pour serrer le dispositif de serrage 35 autour du poignet, les extrémités des parties de sangle 35a, 35b sont engagées dans leurs boucles de serrage respectives 37a, 37b après avoir été enroulées dans un sens autour du poignet. Les parties de sangle 35a, 35b sont ensuite serrées autour du poignet dans l'autre sens, les plaquettes à crochets 36a, 36b aux extrémités libres des parties de sangle 35a, 35b étant appliquées sur la bande à boucles sur le dispositif de serrage 35. L'orthèse peut s'enlever en direction de l'extrémité des doigts et du pouce, sans effort important, notamment grâce à la fente 33 qui élimine la zone d'étranglement autour du poignet.

La figure 14 illustre un autre exemple de réalisation du dispositif de serrage de l'orthèse 3, constitué par une sangle 35c. Une extrémité de la sangle 35c est fixée sur la partie principale 30 de l'orthèse 3, par exemple sur une zone couvrant la face dorsale de la main. Pour serrer la sangle 35c autour du poignet, la sangle 35c est enroulée dans un sens autour de la main, puis engagée dans une boucle de serrage 37c par exemple fixée sur la partie principale 30 sur la zone couvrant la face dorsale du poignet. La sangle 35c est ensuite serrée autour du poignet dans l'autre sens, l'extrémité libre de la sangle 35c étant munie d'une plaquette à crochets 36c prévue pour venir se fixer sur une bande à boucles 31 fixée sur la partie principale 30, dans la région de l'avant-bras. Ce mode d'enroulement de la sangle de serrage 35c qui présente une largeur de l'ordre de 3 cm (à + ou -20% près), permet de couvrir une surface plus importante que le produit du nombre de tours de la sangle par la largeur de cette dernière. Il peut être observé également que ce mode d'enroulement ne recouvre pas la styloïde.

Une baleine 35d peut être fixée entre deux couches de tissu entre l'extrémité de la sangle 35c fixée à la partie principale 30 et la zone de fixation de la boucle 37c sur la partie principale. La baleine 35d permet de rigidifier cette région de la partie principale 30 qui est soumise à des forces de directions opposées lorsque la sangle 35c est serrée. Il peut être observé que la baleine 35d n'est pas recouverte par la sangle 35c, et donc ne peut pas être serrée entre la face dorsale de main et la sangle, et est positionnée sur la face dorsale de la main qui n'est pas amenée à se déformer par un mouvement quelconque de la main ou des doigts. De ce fait, la baleine ne participe pas à rigidifier l'orthèse en complément des plaquettes 32a, 32b.

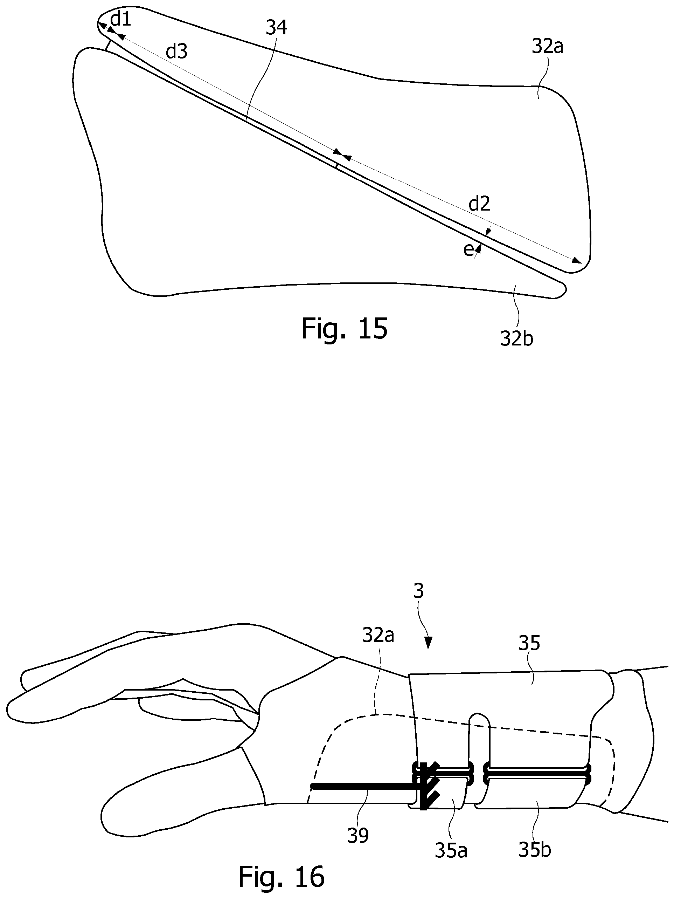

La figure 15 représente la face externe des plaquettes 32a, 32b liées entre elles par une articulation 34. Les plaquettes 32a, 32b présentent ensemble une forme enveloppante, en entourant plus de 50% de la circonférence de la main, du poignet et de l'avant-bras. Cette forme enveloppante présente une profondeur (cote "a" sur la figure 2) qui contribue à augmenter le moment quadratique des plaquettes, et donc, par effet de poutre à augmenter la rigidité des plaquettes pour supporter le poids de la main.

Comme illustré sur la figure 16, une partie des plaquettes 32a, 32b se trouve sous la sangle 35. Les plaquettes 32a, 32b se comportent donc comme des poutres encastrées 39 s'étendant entre le dispositif de serrage 35, 35c et l'extrémité distale du métacarpien du pouce, lorsque la sangle 35, 35c est serrée autour du poignet, le serrage des parties des plaquettes 32a, 32b recouvertes par la sangle 35, 35c produisant un effet d'encastrement. Cet effet d'encastrement est augmenté par le fait que la sangle n'est pas élastique. Ainsi, la forme enveloppante des plaquettes 32a, 32b, associée à la rigidité du matériau choisi pour former les plaquettes, permet maintenir le poignet en position de repos, quelle que soit l'orientation de la main dans l'espace, sans toutefois empêcher les mouvements volontaires du poignet. La rigidité du matériau formant les plaquettes 32a, 32b est choisie de manière à empêcher des mouvements involontaires de l'articulation du poignet à partir de la position de repos neutre de la main, liés au poids de celle-ci, quelle que soit son orientation dans l'espace, sans toutefois empêcher les mouvements volontaires de la main.

Il peut être observé qu'une partie de la charnière 34 se trouve couverte par la sangle de serrage 35, 35c, ce qui contribue à la rigidité de l'orthèse 3. Il peut également être observé que la charnière 34 ne s'étend que sur une partie des bords en regard des deux plaquettes 32a, 32b. Ainsi, la charnière 34 se trouve à des distances d1 et d2 des bords distal et proximal de la plaquette 32a. La distance d2 (figure 15) est fixée de manière à permettre à l'orthèse de s'adapter aux différentes morphologies de la région de l'avant-bras. Cette disposition combinée à la souplesse des plaquettes 32a, 32b permet d'utiliser les mêmes plaquettes pour différentes tailles de l'orthèse 3, et ainsi d'utiliser un seul moule pour la fabrication d'orthèses de différentes tailles. Une couture entre les pièces 10 et 11 formant la poche peut être réalisée dans l'une et/ou l'autre des fentes sans charnière entre les plaquettes 32a, 32b.

La présence de la charnière 34 liant les deux plaquettes 32a, 32b dans la zone d3 permet de former un profil combiné plus profond (cote "a" sur la figure 2) dont la profondeur est augmentée par le serrage de la sangle. Le moment quadratique obtenu par la combinaison des deux plaquettes est donc bien plus élevé dans cette zone. Il peut être observé que cette zone couvre la région du canal carpien où l'on cherche à éviter le contact avec l'orthèse et donc où l'orthèse doit présenter une rigidité plus élevée. Compte tenu de la raideur des plaquettes conférée par leur moment quadratique, les plaquettes peuvent être formées avec une épaisseur réduite.

Selon un exemple de réalisation, la distance d2 entre la charnière 34 et le bord proximal de la plaquette 32a est fixée à une valeur comprise entre 65 et 75 mm, la longueur d3 de la charnière 34 est fixée à une valeur comprise entre 65 et 75 mm, et la distance d1 entre la charnière et le bord distal de la plaquette 32a est fixée à une valeur comprise entre 5 et 10 mm.

Selon un autre mode de réalisation, les plaquettes 32a, 32b ne sont pas liées entre elles par une articulation, mais placées dans deux poches distinctes ou une seule poche divisée en deux, par exemple par une couture de séparation entre les deux plaquettes.

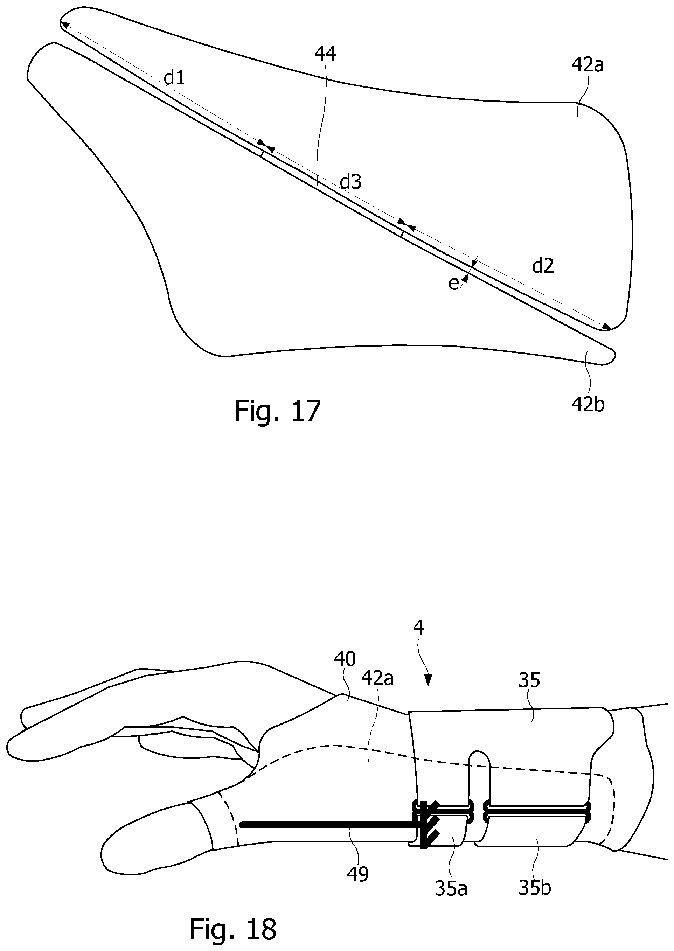

Un autre mode de réalisation concerne une orthèse de poignet et de pouce qui peut combiner les formes des orthèses 2 et 3, en utilisant un dispositif de serrage tel que les dispositifs de serrage 35 et 35c prévus pour l'orthèse 3. La figure 17 représente la face externe de plaquettes 42a, 42b susceptibles d'équiper une telle orthèse de poignet et de pouce. Les plaquettes 42a, 42b peuvent résulter d'une combinaison respectivement de la plaquette 32a avec la plaquette 22a, et de la plaquette 32b avec la plaquette 22b. Les deux plaquettes 42a, 42b sont liées entre elles par une charnière 44 ayant une longueur d3. La charnière est située à une distance d1 du bord distal de la plaquette 42a, et à une distance d2 du bord proximal de la plaquette 42a.

La figure 18 représente une orthèse 4 de poignet et de pouce, équipée des plaquettes 42a, 42b, et serrée autour de la main et du poignet à l'aide du dispositif de serrage 35. Bien entendu, la sangle 35c peut également être utilisée pour serrer l'orthèse 4 autour du poignet. Les plaquettes 42a, 42b se comportent comme des poutres encastrées 49 s'étendant entre le dispositif de serrage 35, 35c et la première phalange du pouce, lorsque le dispositif de serrage 35, 35c est serré autour du poignet, le serrage d'une partie des plaquettes 42a, 42b par le dispositif de serrage 35, 35c produisant un effet d'encastrement. Cet effet d'encastrement est augmenté par le fait que le dispositif de serrage 35, 35c n'est pas élastique. Ainsi, la forme enveloppante des plaquettes 42a, 42b, associée à la rigidité du matériau choisi pour les plaquettes, permet maintenir le poignet et le pouce dans leurs positions de repos respectives, quelle que soit l'orientation de la main dans l'espace, sans toutefois empêcher des mouvements volontaires du pouce et du poignet, à partir de leurs positions de repos neutres respectives, liées à leurs poids respectifs.

Il peut être observé qu'une partie de la charnière 44 se trouve couverte par le dispositif de serrage 35, 35c, ce qui contribue à la rigidité de l'orthèse 4. Il peut également être observé que la charnière 44 ne s'étend que sur une partie des bords en regard des deux plaquettes 42a, 42b. Ainsi, la charnière 44 se trouve à des distances d1 et d2 des bords distal et proximal de la plaquette 42a. Les distances d1 et d2 (figure 17) sont fixées de manière à permettre à l'orthèse de s'adapter aux différentes morphologies de la région du pouce et de l'avant-bras. Cette disposition combinée à la souplesse des plaquettes 42a, 42b permet d'utiliser les mêmes plaquettes pour différentes tailles de l'orthèse 4, et ainsi d'utiliser un seul moule pour la fabrication d'orthèses de différentes tailles. Une couture entre les pièces 10 et 11 formant la poche peut être formée dans l'une et/ou l'autre des fentes sans charnière entre les plaquettes 42a, 42b.

Selon un exemple de réalisation, la distance d2 entre la charnière 44 et le bord proximal de la plaquette 42a est fixée à une valeur comprise entre 65 et 75 mm, la longueur d3 de la charnière 44 est fixée à une valeur comprise entre 45 et 55 mm, et la distance d1 entre la charnière et le bord distal de la plaquette 32a est fixée à une valeur comprise entre 45 et 55 mm.

Selon un autre mode de réalisation, les plaquettes 42a, 42b ne sont pas liées entre elles par une articulation, mais placées dans deux poches distinctes ou une seule poche divisée en deux, par exemple par une couture de séparation entre les deux plaquettes.

Il peut être observé sur les orthèses des figures 7, 8, 10 ,11, 12, 14, 16 et 18, que les plaquettes s'étendent sur plus de 85% de la longueur de l'orthèse le long du membre ou de l'articulation, et enveloppent l'articulation sur plus de 30% (de préférence 50%) du périmètre de la section de celle-ci. La rigidité de l'orthèse dans le sens d'une flexion de l'articulation est définie par l'élasticité du matériau formant les plaquettes et l'épaisseur de celles-ci, par la forme plus ou moins enveloppante des plaquettes, par la présence d'une charnière, et par la rigidité de l'encastrement des plaquettes produit par le dispositif de serrage. Il peut être noté que l'effet de poutre encastrée se produit non seulement en raison de la non élasticité du dispositif de serrage mais également parce que ce dernier ne couvre pas la partie distale des plaquettes.

Il peut être également observé que le tissu formant l'orthèse participe également à la rigidité de l'orthèse, mais avec un ordre de grandeur inférieur à celui des plaquettes.

Pour tenir compte des variations de morphologie d'une personne à une autre, il peut être prévu de fabriquer chaque type d'orthèse précédemment décrite en plusieurs tailles, par exemple 3 ou 4 tailles différentes. Sachant que le poids de la main représente environ 0,5 à 0,7% du poids du corps humain, quelle que soit sa morphologie, le poids maximum à supporter pour chaque taille d'orthèse peut être parfaitement identifié. La rigidité à prévoir pour la plaquette peut donc être déterminée pour chacune des différentes tailles d'orthèse, compte tenu du moment quadratique conféré par la plaquette en raison de sa forme allongée et enveloppante et de l'effet de poutre encastré obtenu grâce au dispositif de serrage opérant seulement dans la région proximale et éventuellement centrale de l'orthèse. Le fait pour le dispositif de serrage de ne pas agir sur la partie distale de l'orthèse permet de conserver une certaine liberté de mouvement pour l'articulation notamment dans des directions non entravées par la plaquette (de la face convexe vers la face concave de la plaquette).

Il apparaîtra clairement à l'homme de l'art que la présente invention est susceptible de diverses variantes de réalisation et diverses applications. En particulier, selon la morphologie de la région de membre recouverte, l'orthèse peut comprendre une seule plaquette formée en une seule partie sans charnière. Il peut également ne pas être nécessaire de mouler la plaquette à la forme du membre à recouvrir. En effet, selon l'articulation et la pathologie à soulager, il peut être suffisant de choisir une plaquette en un matériau suffisamment souple pour épouser naturellement la forme du membre recouverte, sous l'effet du serrage opéré par la pièce 10 de l'orthèse et du dispositif de serrage.

Il n'est pas non plus nécessaire de former la pièce 10 de la partie principale 20, 30 ,40 à l'aide de deux couches de tissu élastique collées ensemble. D'autres matériaux peuvent être aisément trouvés pour donner une rigidité suffisante à la partie principale de l'orthèse. En outre, d'autres matériaux que des tissus peuvent être utilisés pour réaliser le manchon et la poche, tels que des films ou membranes microporeux assemblés par des soudures.

## Revendications

1. Orthèse pour le maintien d'une articulation, adaptée au pouce et/ou au poignet, et comprenant :
une pièce principale (10, 20, 30, 40) en forme de manchon en un matériau élastique, et ajustée pour envelopper l'articulation et des parties de membre distale et proximale de part et d'autre de l'articulation, la pièce principale comportant une ouverture pour le passage d'une partie proximale de membre et une ouverture pour le passage d'une partie distale de membre,
une plaquette (12, 12a, 12b, 22a, 22b, 32a, 32b, 42a, 42b) déformable élastiquement logée dans une poche formée dans la pièce principale et ajustée aux dimensions de la plaquette, une partie distale de la plaquette étant maintenue autour du membre seulement par la pièce principale, la plaquette étant configurée pour que, sur une région d'au moins 50% de la longueur de la pièce principale le long des parties de membre, la plaquette présente une section occupant plus de 30% du périmètre du membre sous-jacent,
**caractérisée en ce qu'**elle comprend un dispositif de serrage (25, 35, 35c) non élastique de la pièce principale dans la région d'au moins 50% de la longueur de la pièce principale, et d'une partie proximale de la plaquette autour de la partie de membre proximale, la plaquette étant réalisée dans un matériau présentant un module de Young compris entre 5 à 10 MPa, tandis que le matériau formant la partie principale présente une rigidité d'un ordre de grandeur inférieur.

2. Orthèse selon la revendication 1, dans laquelle l'étendue et l'épaisseur de la plaquette (22a, 22b, 32a, 32b, 42a, 42b), et l'élasticité de la plaquette et de la partie principale sont choisies de manière à empêcher des mouvements involontaires de l'articulation à partir d'une position de repos neutre, provoqués par le poids de la partie distale de membre.

3. Orthèse selon la revendication 1 ou 2, dans laquelle la plaquette (22a, 22b, 32a, 32b, 42a, 42b) s'étend sur au moins 80% de la longueur de l'orthèse le long du membre, et la section de la plaquette occupe plus de 40% du périmètre du membre sous-jacent sur au moins 80% de la longueur de la plaquette le long du membre.

4. Orthèse selon l'une des revendications 1 à 3, dans laquelle la plaquette comprend deux parties de plaquette (12a, 12b, 22a, 22b, 32a, 32b, 42a, 42b) liées entre elles par une charnière (14, 141, 24, 34, 44) s'étendant le long d'une partie de bords en regard des deux parties de plaquette.

5. Orthèse selon la revendication 4, dans laquelle la charnière (14, 141, 24, 34, 44) est réalisée lors de la fabrication de la plaquette dans le même matériau que la plaquette, ou bien est formée par des points de couture (141, 24).

6. Orthèse selon la revendication 4 ou 5, dans laquelle la charnière (24, 34, 44) s'étend sur moins la moitié de la longueur des bords en regard des deux parties de plaquette (22a, 22b, 32a, 32b, 42a, 42b).

7. Orthèse selon l'une des revendications 4 à 6, dans laquelle le dispositif de serrage (25, 35, 35c) couvre en partie la charnière (24, 34, 44).

8. Orthèse selon la revendication 1, comprenant une autre plaquette, les deux plaquettes (12a, 12b, 22a, 22b, 32a, 32b, 42a, 42b) étant logées dans des poches distinctes et contigües.

9. Orthèse selon l'une des revendications 1 à 8, dans laquelle la pièce principale (10, 20, 30, 40) est réalisée dans une pièce de tissu formée de deux couches de tissu élastique assemblées par une couche de colle, chaque poche étant formée à l'aide d'une couche de tissu élastique (11) cousue sur une face intérieure de la pièce principale.

10. Orthèse selon l'une des revendications 1 à 9, dans laquelle la plaquette (12, 12a, 12b, 22a, 22b, 32a, 32b, 42a, 42b) est réalisée dans un matériau présentant un module de Young compris entre 5,4 et 9 MPa, et présente une épaisseur comprise entre 0,8 et 1,9 mm.

11. Orthèse selon l'une des revendications 1 à 10, adaptée au maintien du pouce dans laquelle la pièce principale (20) comportant une partie distale (20a) en forme de manchon conformé pour couvrir la base du pouce, une partie proximale (20c) en forme de manchon conformé pour couvrir le poignet, et une partie intermédiaire (20b) reliant les parties distale et proximales, la plaquette comprenant deux parties de plaquettes (22a, 22b) conformées pour épouser la forme du pouce, de la première phalange et du premier métacarpien du pouce, jusqu'au poignet, sans recouvrir la zone palmaire du poignet, le dispositif de serrage comprenant une sangle (25) fixée à la pièce principale (20) et agencée pour être serrée autour du poignet.

12. Orthèse selon l'une des revendications 1 à 10, adaptée au maintien du poignet, dans laquelle la pièce principale (30) en forme de manchon, est conformée pour couvrir une partie de l'avant-bras, le poignet et les articulations carpo-métacarpiennes du pouce, et une partie proximale des métacarpes des doigts, la pièce principale présentant une ouverture proximale pour le passage de l'avant-bras, et deux ouvertures distales, l'une pour le passage du pouce, et l'autre pour le passage de la paume de la main, la plaquette comprenant deux parties de plaquettes (32a, 32b) conformées pour épouser la base de la paume de la main, la région du métacarpien du pouce, et la face palmaire du poignet, et d'une partie de l'avant-bras, en remontant sur les faces latérales de la main, du poignet et de l'avant-bras, le dispositif de serrage (35) étant agencé pour serrer la pièce principale autour du poignet et de l'avant-bras.

13. Orthèse selon l'une des revendications 1 à 10, adaptée au maintien du poignet et du pouce, dans laquelle la pièce principale (40) comprend une partie distale (40a) en forme de manchon conformé pour couvrir la base du pouce, une partie proximale (40c) en forme de manchon conformé pour couvrir le poignet et une partie de l'avant-bras et les articulations carpo-métacarpiennes du pouce, et une partie intermédiaire (40b) reliant les parties distale et proximale, la plaquette comprenant deux parties de plaquettes (42a, 42b) conformées pour épouser la forme du pouce, de la première phalange et de la région du premier métacarpien du pouce, la base de la paume de la main, et la face palmaire du poignet, et d'une partie de l'avant-bras, en remontant sur les faces latérales de la main, du poignet et de l'avant-bras, le dispositif de serrage (35) étant agencé pour serrer la pièce principale autour du poignet et de l'avant-bras.

14. Orthèse selon la revendication 12 ou 13, dans laquelle l'une des deux parties de plaquettes (32a, 42a) couvrant la base de la paume de la main est conformée pour ne pas serrer la région du canal carpien sur la face palmaire du poignet.

## Patentansprüche

1. Orthese zum Halten eines Gelenks, die auf den Daumen und/oder das Handgelenk abgestimmt ist, umfassend:
ein Hauptstück (10, 20, 30, 40) in Form einer Manschette aus einem elastischen Material und das zum Umhüllen des Gelenks und des distalen und des proximalen Gliedmaßenteils auf beiden Seiten des Gelenks angepasst ist, wobei das Hauptstück eine Öffnung für den Durchgang eines proximalen Gliedmaßenteils und eine Öffnung für den Durchgang eines distalen Gliedmaßenteils vorweist,
eine elastisch verformbare Scheibe (12, 12a, 12b, 22a, 22b, 32a, 32b, 42a, 42b), die in einer Tasche untergebracht ist, die in dem Hauptstück ausgebildet ist und an die Abmessungen der Scheibe angepasst ist, wobei ein distaler Abschnitt der Scheibe nur durch das Hauptstück um die Gliedmaße herum gehalten wird, wobei die Scheibe so konfiguriert ist, dass die Scheibe über einen Bereich von mindestens 50 % der Länge des Hauptstücks entlang der Gliedmaßenteile einen Abschnitt aufweist, der mehr als 30 % des Umfangs der darunterliegenden Gliedmaße einnimmt,
**dadurch gekennzeichnet, dass** sie eine nicht elastische Klemmvorrichtung (25, 35, 35c) des Hauptstücks in dem Bereich von mindestens 50 % der Länge des Hauptstücks und eines proximalen Teils der Scheibe um das proximale Gliedmaßenteil herum umfasst, wobei die Scheibe aus einem Material hergestellt ist, das einen Elastizitätsmodul zwischen 5 und 10 MPa aufweist, während das Material, das das Hauptstück ausbildet, eine um eine Größenordnung geringere Steifigkeit aufweist.

2. Orthese nach Anspruch 1, wobei die Ausdehnung und die Dicke der Scheibe (22a, 22b, 32a, 32b, 42a, 42b) und die Elastizität der Scheibe und des Hauptstücks so gewählt sind, dass unbeabsichtigte Bewegungen des Gelenks aus einer neutralen Ruheposition, verursacht durch das Gewicht des distalen Gliedmaßenteils, verhindert werden.

3. Orthese nach Anspruch 1 oder 2, wobei sich die Scheibe (22a, 22b, 32a, 32b, 42a, 42b) über mindestens 80 % der Länge der Orthese entlang der Gliedmaße erstreckt und der Abschnitt der Scheibe über mindestens 80 % der Länge der Scheibe entlang der Gliedmaße mehr als 40 % des Umfangs der darunterliegenden Gliedmaße einnimmt.

4. Orthese nach einem der Ansprüche 1 bis 3, wobei die Scheibe zwei Scheibenteile (12a, 12b, 22a, 22b, 32a, 32b, 42a, 42b) umfasst, die durch ein Scharnier (14, 141, 24, 34, 44) miteinander verbunden sind, das sich entlang eines Teils von Kanten gegenüber den zwei Scheibenteilen erstreckt.

5. Orthese nach Anspruch 4, wobei das Scharnier (14, 141, 24, 34, 44) bei der Anfertigung der Scheibe aus dem gleichen Material wie die Scheibe hergestellt oder aber durch Nadelstiche (141, 24) ausgebildet wird.

6. Orthese nach Anspruch 4 oder 5, wobei sich das Scharnier (24, 34, 44) über weniger als die Hälfte der Länge der Kanten gegenüber den zwei Scheibenteilen (22a, 22b, 32a, 32b, 42a, 42b) erstreckt.

7. Orthese nach einem der Ansprüche 4 bis 6, wobei die Klemmvorrichtung (25, 35, 35c) das Scharnier (24, 34, 44) teilweise bedeckt.

8. Orthese nach Anspruch 1, umfassend eine weitere Scheibe, wobei die zwei Scheiben (12a, 12b, 22a, 22b, 32a, 32b, 42a, 42b) in getrennten und aneinandergrenzenden Taschen untergebracht sind.

9. Orthese nach einem der Ansprüche 1 bis 8, wobei das Hauptstück (10, 20, 30, 40) aus einem Gewebestück hergestellt ist, das aus zwei Schichten elastischen Gewebes ausgebildet ist, die durch eine Schicht Klebstoff zusammengefügt sind, wobei jede Tasche mithilfe einer Schicht elastischen Gewebes (11) ausgebildet ist, die auf eine Innenfläche des Hauptstücks genäht ist.

10. Orthese nach einem der Ansprüche 1 bis 9, wobei die Scheibe (12, 12a, 12b, 22a, 22b, 32a, 32b, 42a, 42b) aus einem Material hergestellt ist, das einen Elastizitätsmodul zwischen 5,4 und 9 MPa aufweist und eine Dicke zwischen 0,8 und 1,9 mm aufweist.

11. Orthese nach einem der Ansprüche 1 bis 10, die zum Halten des Daumens abgestimmt ist, wobei das Hauptstück (20) einen distalen Teil (20a) in Form einer Manschette, die zum Bedecken der Daumenbasis geformt ist, einen proximalen Teil (20c) in Form einer Manschette, die zum Bedecken des Handgelenks geformt ist, und einen Zwischenteil (20b) vorweist, der den distalen und den proximalen Teil verbindet, die Scheibe umfassend zwei Scheibenteile (22a, 22b) die zum Anschmiegen an die Form des Daumens, der ersten Phalanx und des ersten Mittelhandknochens des Daumens bis zu dem Handgelenk geformt sind, ohne die Hohlhandzone des Handgelenks zu überdecken, die Klemmvorrichtung umfassend einen Riemen (25), der an dem Hauptstück (20) befestigt ist und zum Klemmen um das Handgelenk herum angeordnet ist.

12. Orthese nach einem der Ansprüche 1 bis 10, die zum Halten des Handgelenks abgestimmt ist, wobei das Hauptstück (30) in Form einer Manschette zum Bedecken eines Teils des Unterarms, des Handgelenks und der Daumensattelgelenke und eines proximalen Teils der Mittelhandknochen der Finger geformt ist, wobei das Hauptstück eine proximale Öffnung für den Durchgang des Unterarms und zwei distale Öffnungen aufweist, die eine für den Durchgang des Daumens und die andere für den Durchgang der Handfläche, die Scheibe umfassend zwei Scheibenteile (32a, 32b), die zum Anschmiegen an die Basis der Handfläche, den Bereich des Mittelhandknochens des Daumens und die Hohlhandfläche des Handgelenks und eines Teils des Unterarms geformt ist, nach oben an den Seitenflächen der Hand, des Handgelenks und des Unterarms, wobei die Klemmvorrichtung (35) zum Festklemmen des Hauptstücks um das Handgelenk und den Unterarm herum angeordnet ist.

13. Orthese nach einem der Ansprüche 1 bis 10, die zum Halten des Handgelenks und des Daumens abgestimmt ist, wobei das Hauptstück (40) einen distalen Teil (40a) in Form einer Manschette, die zum Bedecken der Daumenbasis geformt ist, einen proximalen Teil (40c) in Form einer Manschette, die zum Bedecken des Handgelenks und eines Teils des Unterarms und der Daumensattelgelenke geformt ist, und einen Zwischenteil (40b) umfasst, der den distalen und den proximalen Teil verbindet, die Scheibe umfassend zwei Scheibenteile (42a, 42b), die zum Anschmiegen an die Form des Daumens, der ersten Phalanx und des Bereichs des ersten Mittelhandknochens des Daumens, der Basis der Handfläche und der Hohlhandfläche des Handgelenks und eines Teils des Unterarms geformt sind, nach oben an den Seitenflächen der Hand, des Handgelenks und des Unterarms, wobei die Klemmvorrichtung (35) zum Festklemmen des Hauptstücks um das Handgelenk und den Unterarm herum angeordnet ist.

14. Orthese nach Anspruch 12 oder 13, wobei der eine der zwei Scheibenabschnitte (32a, 42a), die die Basis der Handfläche bedecken, so geformt ist, dass er den Karpaltunnelbereich auf der Handfläche des Handgelenks nicht festklemmt.

## Claims

1. An orthosis for supporting a joint, adapted to the thumb and/or the wrist, comprising:
a sleeve-like main member (10, 20, 30, 40) of an elastic material, and adapted to envelop the joint and distal and proximal limb portions on either side of the joint, the main member having an opening for the passage of a proximal limb portion and an opening for the passage of a distal limb portion,
an elastically deformable panel (12, 12a, 12b, 22a, 22b, 32a, 32b, 42a, 42b) housed in a pocket formed in the main member and adjusted to the dimensions of the panel, and
a distal portion of the panel being held around the limb only by the main member,
the panel being configured so that, over a region of at least 50% of the length of the main member along the limb portions, the panel has a cross-section occupying more than 30% of the perimeter of the underlying limb,
**characterized in that** it comprises a rigid bracing device (25, 35, 35c) for bracing the main member over a regions of at least 50% of the length of the main member and a proximal portion of the panel around the proximal limb portion, the panel being made of a material having a Young's modulus between 5 and 10 MPa, while the material forming the main member has a rigidity of a lower order of magnitude.

2. The orthosis according to claim 1, wherein the extent and thickness of the panel (22a, 22b, 32a, 32b, 42a, 42b), and the elasticity of the panel and the main member are selected so as to prevent unintentional movements of the joint from a neutral rest position due to the weight of the distal limb part.

3. The orthosis according to claim 1 or 2, wherein the panel (22a, 22b, 32a, 32b, 42a, 42b) extends over at least 80% of the length of the orthosis along the limb, and the cross-section of the panel occupies more than 40% of the perimeter of the underlying limb over at least 80% of the length of the panel along the limb.

4. The orthosis according to one of claims 1 to 3, wherein the panel comprises two panel parts (12a, 12b, 22a, 22b, 32a, 32b, 42a, 42b) joined together by a hinge (14, 141, 24, 34, 44) extending along part of facing edges of the two panel parts.

5. The orthosis according to claim 4, wherein the hinge (14, 141, 24, 34, 44) is made during the manufacture of the panel from the same material as the panel or is formed by stitches (141, 24).

6. The orthosis according to claim 4 or 5, wherein the hinge (24, 34, 44) extends over less than half the length of the facing edges of the two panel portions (22a, 22b, 32a, 32b, 42a, 42b).

7. The orthosis according to one of claims 4 to 6, in which the bracing device (25, 35, 35c) partly covers the hinge (24, 34, 44).

8. The orthosis according to claim 1, comprising another panel, the two panels (22a, 22b, 32a, 32b, 42a, 42b) being housed in distinct and contiguous pockets.

9. The orthosis according to one of claims 1 to 8, wherein the main member (10, 20, 30, 40) is made of fabric including two elastic fabric layers joined together by an adhesive layer, each pocket being formed by means of an elastic fabric layer (11) sewn onto an inner side of the main member.

10. The orthosis according to one of claims 1 to 9, wherein the panel (12, 12a, 12b, 22a, 22b, 32a, 32b, 42a, 42b) is made of a material having a Young's modulus between 5.4 and 9 MPa, and has a thickness between 0.8 and 1.9 mm.

11. The orthosis according to one of claims 1 to 10, adapted to support the thumb, wherein the main member (20) includes a distal sleeve-like part (20a) shaped to cover the base of the thumb, a proximal sleeve-like part (20c) shaped to cover the wrist, and an intermediate part (20b) connecting the distal and proximal parts, the panel comprising two panel parts (22a, 22b) shaped to conform to the shape of the thumb, the first phalanx and the first metacarpal of the thumb, up to the wrist, without covering the palmar area of the wrist, the bracing device comprising a strap (25) attached to the main member (20) and configured to be tightened around the wrist.

12. The orthosis according to one of claims 1 to 10, adapted to support the wrist, wherein the sleeve-like main member (30) is shaped to cover a portion of the forearm, the wrist and the carpo-metacarpal joints of the thumb and a proximal portion of the metacarpals of the fingers, the main member having a proximal opening for passage of the forearm and two distal openings, one for passage of the thumb, and the other for the passage of the palm of the hand, the panel comprising two panel parts (32a, 32b) shaped to conform to the base of the palm of the hand, the metacarpal region of the thumb, and the palmar surface of the wrist, and part of the forearm, extending up along the side faces of the hand, wrist and forearm, the bracing device (35) being configured to brace the main member around the wrist and forearm.

13. The orthosis according to one of claims 1 to 10, adapted to support the wrist and thumb, wherein the main member (40) includes a distal sleeve-like part (40a) shaped to cover the base of the thumb, a proximal sleeve-like part (40c) shaped to cover the wrist and part of the forearm and the carpo-metacarpal joints of the thumb, and an intermediate part (40b) connecting the distal and proximal parts, the panel including two panel parts (42a, 42b) shaped to conform to the shape of the thumb, the first phalanx and the region of the first metacarpal of the thumb, the base of the palm of the hand, and the palmar surface of the wrist, and part of the forearm, extending up along the side faces of the hand, wrist and forearm, the bracing device (35) being configured to brace the main member around the wrist and forearm.

14. The orthosis according to claim 12 or 13, wherein one of the two panel parts (32a, 42a) covering the base of the palm of the hand is shaped so as not to tighten the carpal tunnel region on the palmar surface of the wrist.
